# EUROPÄISCHE PATENTSCHRIFT

(11) **EP 1 507 524 B1**
(45) Veröffentlichungstag und Bekanntmachung des Hinweises auf die Patenterteilung: **10.01.2007**
(21) Anmeldenummer: 03732483.7
(22) Anmeldetag: 27.05.2003
(51) Int. Cl.: A61K 31/196, A61K 31/40, A61K 31/341, A61K 31/41, A61K 31/44, A61P 17/00

(54) **TOPISCHE VERWENDUNG VON DIURETIKA ZUR BEHANDLUNG VON SCHWELLUNGEN**
TOPICAL USE OF DIURETICS FOR TREATING SWELLINGS
UTILISATION TOPICALE DE DIURETIQUES POUR TRAITER LES OEDEMES

(30) Priorität: 29.05.2002 DE 10223932
(43) Veröffentlichungstag der Anmeldung: 23.02.2005
(73) Patentinhaber: Wank, Rudolf, 80469 München (DE)
(72) Erfinder: Wank, Rudolf, 80469 München (DE)
(74) Vertreter: Wibbelmann, Jobst
(86) Internationale Anmeldenummer: PCT/EP2003/005575
(87) Internationale Veröffentlichungsnummer: WO 2003/099271

(56) Entgegenhaltungen:
- EP-A- 0 247 507
- WO-A-01/49242
- WO-A-96/15771
- WO-A-96/17602
- BEHRENDT P ET AL: "[Diagnosis and therapy of diseases of the lymph vessels]" DER INTERNIST. GERMANY JAN 2002, Bd. 43, Nr. 1, Januar 2002 (2002-01), Seiten 57-67, XP002254030 ISSN: 0020-9554
- WHITE S D ET AL: "ACQUIRED CUTANEOUS LYMPHANGIECTASIS IN A DOG" JOURNAL OF THE AMERICAN VETERINARY MEDICAL ASSOCIATION, Bd. 193, Nr. 9, 1988, Seiten 1093-1094, XP009016953 ISSN: 0003-1488

## Beschreibung

Die vorliegende Erfindung betrifft topische Präparationen, die Diuretika enthalten, insbesondere Schleifendiuretika, sowie deren Verwendung zur Behandlung von lokalen Schwellungen der Haut.

Als Diuretika werden Substanzen bezeichnet, die eine vermehrte Wasser- und Salzausscheidung verursachen. Ihr Anwendungsgebiet umfasst sowohl die Behandlung von Nierenerkrankungen als auch die Behandlung von arterieller Hypertonie, Herzinsufflzienz und Ausschwemmungen von allgemeinen oder Organödemen. Diuretika werden je nach Wirkprinzip in unterschiedliche Gruppen unterteilt, zu denen osmotische Diuretika, Carboanhydrase-Hemmstoffe, Thiazid-Diuretika, Schleifendiuretika, Kalium-sparenden Diuretika und Aldosteron-Antagonisten gehören.

Schleifendiuretika werden insbesondere bei der Behandlung von Organödemen (insbesondere kardial, renal oder hepatisch bedingte Ödeme) und als Antihypertensiva eingesetzt. Sie zeichnen sich durch einen Hemmeffekt auf die Chlorid- und Natrium-Rückresorption im aufsteigenden Schenkel der Henle-Schleife aus und wirken auch bei reduzierter glomerulärer Filtrationsrate. Einer der prominentesten Vertreter der Schleifendiuretika ist Furosemid, des weiteren zählen Azosemid, Bumetanid, Piretanid und Torasemid zur Gruppe der Schleifendiuretika. Schleifendiuretika werden peroral oder intravenös verabreicht und finden sowohl bei Kurzzeit- als auch bei Langzeit-Therapien eine Anwendung. In der EP 0 247 507 wird ein transdermales therapeutisches System für Schleifendiuretika beschrieben, das über perkutane Resorption eine retardierte systemische Wirkung erzeugt.

Es wurde nun überraschend gefunden, dass topische pharmazeutische Präparationen von Diuretika, insbesondere Schleifendiuretika, zur lokalen Behandlung von Schwellungen der Haut geeignet sind. Sie können die intravenöse Infusion von Schleifendiuretika an Wirkung sogar deutlich übertreffen oder die Wirkung von vorher wirkungslosen intravenösen Infusionen von Schleifendiuretika ermöglichen. Dadurch werden lokale Ödeme therapierbar, die bisher durch manuelle Drainage nur unzureichend behandelt werden konnten.

Die Erfindung betrifft daher die Verwendung eines Diuretikums, insbesondere eines Schleifendiuretikums, oder eines pharmazeutisch annehmbaren Salzes davon zur Herstellung einer topischen pharmazeutischen Präparation zur Behandlung von lokalen Schwellungen der Haut, die durch eine Venenentzündung oder eine lymphatische Entzündung verursacht wurden.

Im Rahmen der vorliegenden Erfindung können grundsätzlich alle oben genannten Diuretika-Arten verwendet werden. Dazu gehören beispielsweise die Thiazid-Diuretika, wie Hydrochlorothiazid (6-Chlor-3,4-dihydro-2*H*-1,2,4-benzothiadiazin-7-sulfonamid-1,1-dioxid) und Etacrynsäure ([2,3-Dichlor-4-(2-ethylacryloyl)-phenoxy]-essigsäure), die Carboanhydrase-Hemmstoffe, wie Acetazolamid (5-Acetylamino-1,3,4-thiadiazol-2-sulfonamid), die Kalium-sparenden Diuretika, wie Triamteren (2,4,7-Triamino-6-phenylpteridin), und insbesondere die Schleifendiuretika.

Zu den Schleifendiuretika, wie sie im Rahmen der vorliegenden Erfindung eingesetzt werden können, gehören Furosemid (4-Chlor-N-furfuryl-5-sulfoamoyl-anthranilsäure), Azosemid (2-Chlor-5-(1H-tetrazol-5-yl)-2-thienylsulfanilamid), Bumetanid (3-Butylamino-4-phenoxy-5-sulfamoylbenzoesäure), Piretanid (4-Phenoxy-3-(1-pyrrolidinyl)-5-sulfamoylbenzoesäure) und Torasemid (1-Iso-propyl-3-[(4-toluidino-3-pyridyl)-sulfonyl]urea). Bevorzugt werden Furosemid, Piretanid und Torasemid eingesetzt, ganz besonders bevorzugt Furosemid. Pharmazeutische Präparate dieser Wirkstoffe in Tablettenform oder als Injektionslösungen sind kommerziell erhältlich.

Als pharmazeutisch annehmbare Salze kommen sowohl wasserlösliche wie auch schwerwasserlösliche Salze, die dem Fachmann allgemein bekannt sind, in Betracht. Unter die erfindungsgemäßen topischen Präparationen, die ein oder mehrere Diuretika, insbesondere Schleifendiuretika, als Wirkstoff enthalten, und sich zur Applikation auf der Haut eignen, fallen Salben, Pasten, Lotionen, Cremes, Gele oder auch Tinkturen. Unter Tinkturen im Rahmen der vorliegenden Erfindung fallen solche wirkstoffhaltigen Präparationen in flüssiger Form, die zur Applikation auf der Haut geeignet sind, nicht aber für perorale oder intravenöse Anwendungen bestimmt sind.

Die Herstellung der Präparationen erfolgt in bekannter Weise, wobei die bekannten und üblichen pharmazeutischen Hilfsstoffe sowie sonstige übliche Träger- und Verdünnungsmittel verwendet werden (List et al., Arzneiformenlehre, Wiss. Verlagsges., Stuttgart, 1985; Sucka et al., Pharmazeutische Technologie, Thieme Verlag, Stuttgart, 1978).

Beispielsweise können bei der Herstellung einer Salbe Organogele (z.B. Vaseline, Plastibase), Lipogele (z.B. Bienenwachs), Hydrogele (z.B. Aerosil^{®}, Bentonite, Stärkederivate, Polyacrylsäure, Polyethylenglykole) oder Silicongele als Salbengrundlage verwendet werden. Bevorzugt dient Vaseline als Salbengrundlage, wobei die Salbe noch weitere Materialien wie Fettalkohole, Glycerylmonostearate, Triglyceride, Alkylenglykole, Dimethylsulfoxid und Wasser enthalten kann. Ganz besonders bevorzugt wird als Salbengrundlage die DAC-Basiscreme (Cremor basalis, Deutscher Arzneimittel-Codex) verwendet, die weiße Vaseline, Glycerolmonostearat 60, Cetylalkohol, mittelkettige Triglyceride, Macrogol-1000-glycerolmonostearat, Propylenglykol und Wasser enthält.

Bei der Herstellung von Cremes können die oben angeführten Salbengrundlagen in Kombination mit größeren Mengen Wasser verwendet werden, insbesondere auch Fette und Öle, Wachse, Fettsäuren und Fettsäureester, langkettige Alkohole und Emulgierstoffe.

Zu den Präparaten in Gel-Form gehören Organogele (d.h. Kohlenwasserstoff-gele z.B. Vaseline, Plastibase), Lipogele (z.B. natürliche Fette, Bienenwachs), Hydrogele (z.B. Hydroxypropylcellulose, Hydroxypropylmethylcellulose, Aerosil^{®}, Bentonite, Stärkederivate, Polyacrylsäure, Polyethylenglykole), Silicongele oder Emulsionsgele, die übliche Emulgatoren enthalten wie auch Oleogele, die im wesentlichen aus flüssigem Paraffin mit Polyethylen oder Ölen und Verdickungsmitteln zusammengesetzt sind.

Darüber hinaus ist der Zusatz von Konservierungsmitteln, Stabilisatoren, Puffersubstanzen, Farbstoffen, Antioxidantien usw. möglich.

Zur Herstellung von Tinkturen zur topischen Anwendung auf der Haut kommen beispielsweise Wasser oder physiologisch verträgliche Lösungsmittel wie beispielsweise Alkohole (Ethanol, Propylenglykol oder Polyglykole), Öle oder Paraffine in Frage.

Die erfindungsgemäßen pharmazeutischen topischen Präparationen enthalten das Diuretikum oder ein pharmazeutisch annehmbares Salz davon in einer Menge von 0,1 bis 10 Gew.-%, bezogen auf die pharmazeutische Präparation. Bevorzugt liegt der Wirkstoffgehalt im Bereich von 0,1 - 1 Gew.-%, ganz besonders bevorzugt bei 0.2 Gew.-%.

Zur Einführung der Diuretika in die erfindungsgemäßen topischen Präparationen können handelsübliche Wirkstoffzubereitungen, wie beispielsweise Lösungen in Ampullenform, verwendet werden. Verschiedene Furosemid-Lösungen sind beispielsweise erhältlich bei Hoechst (Lasix^{®}), CT (Furo^{®}), Ratiopharm (Furosemid-Ratiopharm^{®}) und Hexal (Furores^{®}).

Gemäß einer bevorzugten Ausführungsform liegt die pharmazeutische Präparation in Form einer Salbe vor, die Furosemid oder ein pharmazeutisch annehmbares Salz davon in einer Menge von 0,2 Gew.-%, bezogen auf die pharmazeutische Präparation, enthält.

Ohne, dass beabsichtigt wird, auf eine bestimmte Theorie zur Erklärung der feststellbaren Wirkung von Diuretika, insbesondere Schleifendiuretika, festgelegt zu werden, werden die im Folgenden erläuterten Wirkungsmechanismen in Betracht gezogen.

Eine Behinderung des venösen und lymphatischen Abflusses und der resultierende Rückstau in beiden Gefäßsystemen werden als Hauptfaktoren des lokalen Ödems dargestellt.

Schleifendiuretika hemmen Na+K+Cl-Cotransporter. Es gibt mindestens sechs verschiedene Isoformen von absorbierenden Cotransportern. Ein Cotransporter wurde erstmals in einer Analdrüse des Haifisches isoliert, es handelt sich um einen sekretorischen Cotransporter. Während der "absorbierende" Cotransporter nur in der Niere exprimiert zu sein scheint, sind sekretorische Cotransporter in vielen Geweben exprimiert. Es könnte sich also bei dem lokalen Effekt von Furosemid um eine Blockade eines sekretorischen Cotransporters handeln.

In letzter Zeit sind jedoch viele Ergebnisse aufgetaucht, die eine "alleinige" Spezialistenleistung von verschiedenen Geweben relativiert haben. Es konnte gezeigt werden, dass Immunzellen Neurotrophine und Hypophysenhormone produzieren, deren Produktion lange Zeit nur Gehirnzellen zugeschrieben wurde. Jüngst wurde eine invariante Kette des T-Zellrezeptors auf Gehirnzellen entdeckt. Es scheint so, dass ein Rezeptor oder ein Genprodukt zunächst in dem Gewebe mit der höchsten Expression entdeckt wird, also am Ort der intensivsten Nutzung. Andere Gewebe können jedoch mit denselben Molekülen dieselben Aufgaben auf niedrigerem Niveau durchführen.

Von Schleifendiuretika ist jedoch noch ein wichtiger Effekt bekannt, der nicht mit der "klassischen" Anlagerung an die CI-Bindungsstelle des Cotransporterproteins im aufsteigenden Schenkel der Henle-Schleife erklärt werden kann:

Schleifendiuretika erhöhen die venöse Aufnahmekapazität (vasodilatorischer Effekt). Die Wirkungsweise ist nicht bekannt. Die profunde Wirkung der Schleifendiuretika beim Lungenödem setzt vor dem Beginn der Diurese ein und wird auf die Erhöhung der venösen Aufnahmekapazität zurückgeführt.

Somit könnte die lokale Wirkungsweise mit einer Hemmung des sekretorischen Cotransporters und/oder einem vasodilatorischen Effekt in Zusammenhang stehen. Schleifendiuretika können jedoch auch die Na+K+ATPase, die Glykolyse, die mitochondriale Atmung, die mikrosomale Ca2+Pumpe, die Adenylcyclase, die Phosphodieesterase und die Prostaglandin-Dehydrogenase hemmen. Diese früher nur in vitro beobachteten Hemmeffekte der Elektrolyttransporte konnten in vielen Geweben nur bei hohen Konzentrationen beobachtet werden. Möglicherweise können solch hohe Konzentrationen durch die Salbenanwendung lokal erzielt werden.

Die pharmazeutische Präparation in topischer Form wird mehrmals täglich, in der Regel zwei bis vier Mal pro Tag, auf die betroffenen Hautareale aufgetragen. Je nach Bedarf kann die Häufigkeit der Applikation angepasst werden. Die lokale Behandlung einer Schwellung mit der erfindungsgemäßen Präparation kann zusammen mit einer auf die Erkrankung abgestimmten medikamentösen Behandlung erfolgen.

Im Folgenden wird die Erfindung anhand von Beispielen näher erläutert.

### Herstellung einer Furosemid-Salbe

Als Salbengrundlage wurde die Basiscreme DAC (Cremor basalis, Deutscher Arzneimittel-Codex) mit folgender Zusammensetzung eingesetzt:

| auf 100 g Zubereitung: | |
|---|---|
| Glycerolmonostearat 60 | 4,0 g |
| Cetylalkohol | 6,0 g |
| Mittelkettige Triglyceride | 7,5 g |
| Weiße Vaseline | 25,5 g |
| Macrogol-1000-glycerolmonostearat | 7,0 g |
| Propylenglycol | 10,0 g |
| Wasser | 40 g |

Für die Herstellung der Salbe wurden 68 g Basiscreme DAC, 12 g Dimethylsulfoxid und 200 mg Furosemid (20 ml Lasix^{®} Wirkstoffkonzentration 10 mg/ml) eingesetzt.

### Patientenstudien

Die Behandlung eines Krebspatienten mit Schwellung des Genitales nach Leukozytenapharese führte nach lokaler Applikation der Furosemid-Salbe (aus obigem Beispiel) zu einem deutlichen Rückgang der Schwellung innerhalb von wenigen Stunden. Dieser Effekt konnte durch eine vorhergehende intravenöse Verabreichung von Furosemid in Kombination mit Spironolacton nicht erzielt werden, was ein deutliches Indiz für die lokale Wirkung der Furosemid-Salbe ist. Das Furosemid in Salbenform wirkt nur lokal und nicht systemisch, was dazu führt, dass keine Störung des Elektrolythaushalts der Patienten auftritt.

Eine Behandlung eines sogenannten Lympharms bei einer Brustkrebspatientin mit der Furosemid-Salbe (aus obigem Beispiel) führte nach wenigen Anwendungen zu einer deutlichen Reduzierung des Ödems, so dass auf die Durchführung einer Lymphdrainage verzichtet werden konnte.

Bei einer weiteren Patientin konnte eine Schwellung des Unterarms, die auf eine Venenentzündung zurückzuführen war, durch lokale Anwendung der Furosemid-Salbe (aus obigem Beispiel) innerhalb von 12 Stunden deutlich reduziert werden.

Die erfindungsgemäße furosemidhaltige Salbe führte innerhalb von kurzer Zeit zu einem deutlichen Rückgang von Schwellungen ganz unterschiedlicher Natur, und eröffnet somit neue und effiziente Ansätze bei der Behandlung von Schwellungen.

## Patentansprüche

1. Verwendung von einem Diuretikum oder einem pharmazeutisch annehmbaren Salz davon zur Herstellung einer pharmazeutischen Präparation in topischer Form zur Behandlung von lokalen Schwellungen auf der Haut, **dadurch gekennzeichnet, dass** die Schwellungen durch eine Venenentzündung oder eine lymphatische Entzündung verursacht worden sind.

2. Verwendung gemäß Anspruch 1, **dadurch gekennzeichnet, dass** das Diuretikum ein Schleifendiuretikum ist.

3. Verwendung gemäß Anspruch 2, **dadurch gekennzeichnet, dass** das Schleifendiuretikum Furosemid, Azosemid, Bumetanid, Piretanid oder Torasemid oder ein pharmazeutisch annehmbares Salz davon ist.

4. Verwendung gemäß Anspruch 3, **dadurch gekennzeichnet, dass** das Schleifendiuretikum Furosemid oder ein pharmazeutisch annehmbares Salz davon ist.

5. Verwendung gemäß einem der Ansprüche 1 bis 4, **dadurch gekennzeichnet, dass** die pharmazeutische Präparation ein Diuretikum oder ein pharmazeutisch annehmbares Salz davon in einer Menge von 0,1 bis 10 Gew.-%, bezogen auf die pharmazeutische Präparation, enthält.

6. Verwendung gemäß Anspruch 5, **dadurch gekennzeichnet, dass** die pharmazeutische Präparation ein Diuretikum oder ein pharmazeutisch annehmbares Salz davon in einer Menge von 0,2 Gew.-%, bezogen auf die pharmazeutische Präparation, enthält.

7. Verwendung gemäß einem der Ansprüche 1 bis 6, **dadurch gekennzeichnet, dass** die pharmazeutische topische Präparation in Form einer Salbe, Paste, Lotion, Creme, Tinktur oder eines Gels vorliegt.

8. Verwendung gemäß einem der Ansprüche 1 bis 7, **dadurch gekennzeichnet, dass** die pharmazeutische Präparation übliche pharmazeutische Hilfsstoffe, Trägermittel und/oder Verdünnungsmittel enthält.

9. Verwendung gemäß einem der vorhergehenden Ansprüche, **dadurch gekennzeichnet, dass** die pharmazeutische Präparation 2-4 mal täglich lokal auf die betroffenen Hautareale aufgetragen wird.

10. Verwendung gemäß einem der vorhergehenden Ansprüche, **dadurch gekennzeichnet, dass** die pharmazeutische Präparation zusammen mit einer auf die Erkrankung abgestimmten medikamentösen Behandlung erfolgt.

## Claims

1. Use of a diuretic or a pharmaceutically acceptable salt thereof for the manufacture of a pharmaceutical preparation in topical form for the treatment of local skin swellings, **characterized in that** the swellings have been caused by a venous or lymphatic inflammation.

2. Use according to Claim 1, **characterized in that** the diuretic is a loop diuretic.

3. Use according to Claim 2, **characterized in that** the loop diuretic is furosemide, azosemide, bumetanide, piretanide or torasemide or a pharmaceutically acceptable salt thereof.

4. Use according to Claim 3, **characterized in that** the loop diuretic is furosemide or a pharmaceutically acceptable salt thereof.

5. Use according to one of Claims 1 to 4, **characterized in that** the pharmaceutical preparation contains a diuretic or a pharmaceutically acceptable salt thereof in an amount of 0.1 to 10 wt.%, based on the pharmaceutical preparation.

6. Use according to Claim 5, **characterized in that** the pharmaceutical preparation contains a diuretic or a pharmaceutically acceptable salt thereof in an amount of 0.2 wt.%, based on the pharmaceutical preparation.

7. Use according to one of Claims 1 to 6, **characterized in that** the pharmaceutical topical preparation is in the form of an ointment, paste, lotion, cream, tincture or gel.

8. Use according to one of Claims 1 to 7, **characterized in that** the pharmaceutical preparation contains conventional pharmaceutical auxiliary substances, excipients and/or diluents.

9. Use according to one of the preceding claims, **characterized in that** the pharmaceutical preparation is applied locally to the affected areas of skin 2-4 times a day.

10. Use according to one of the preceding claims, **characterized in that** the pharmaceutical preparation takes place together with a medicinal treatment appropriate for the disease.

## Revendications

1. Utilisation d'un diurétique ou d'un sel pharmaceutiquement acceptable de celui-ci pour la production d'une préparation pharmaceutique sous forme topique destinée au traitement des oedèmes locaux de la peau, **caractérisée en ce que** les oedèmes se sont formés à la suite d'une phlébite ou d'une inflammation du système lymphatique.

2. Utilisation selon la revendication 1, **caractérisée en ce que** le diurétique est un diurétique de l'anse.

3. Utilisation selon la revendication 2, **caractérisée en ce que** le diurétique de l'anse est un furosémide, un azosémide, un bumétanide, un pirétanide ou un torasémide ou un sel pharmaceutiquement acceptable de ces derniers.

4. Utilisation selon la revendication 3, **caractérisée en ce que** le diurétique de l'anse est un furosémide ou un sel pharmaceutiquement acceptable de celui-ci.

5. Utilisation selon l'une des revendications 1 à 4, **caractérisée en ce que** la préparation pharmaceutique contient un diurétique ou un sel pharmaceutiquement acceptable de celui-ci dans une quantité de 0,1 à 10% en poids par rapport à la préparation pharmaceutique.

6. Utilisation selon la revendication 5, **caractérisée en ce que** la préparation pharmaceutique contient un diurétique ou un sel pharmaceutiquement acceptable de celui-ci dans une quantité de 0,2% en poids par rapport à la préparation pharmaceutique.

7. Utilisation selon l'une des revendications 1 à 6, **caractérisée en ce que** la préparation pharmaceutique topique se présente sous la forme d'une pommade, pâte, lotion, crème, teinture ou d'un gel.

8. Utilisation selon l'une des revendications 1 à 7, **caractérisée en ce que** la préparation pharmaceutique contient des adjuvants, des substances porteuses et/ou des diluants pharmaceutiques courants.

9. Utilisation selon l'une des revendications précédentes, **caractérisée en ce que** la préparation pharmaceutique est appliquée localement 2 à 4 fois par jour sur les zones cutanées concernées.

10. Utilisation selon l'une des revendications précédentes, **caractérisée en ce que** l'application locale de la préparation pharmaceutique est effectuée conjointement avec un traitement médicamenteux adapté à l'affection.
